# EUROPÄISCHE PATENTSCHRIFT

(11) **EP 3 258 889 B1**
(45) Veröffentlichungstag und Bekanntmachung des Hinweises auf die Patenterteilung: **09.10.2024**
(21) Anmeldenummer: 16711302.6
(22) Anmeldetag: 23.03.2016
(51) Int. Cl.: A61F 2/50

(54) **ORTHESE UND VERFAHREN ZUR HERSTELLUNG EINER ORTHESE**
ORTHOSIS AND METHOD FOR PRODUCING AN ORTHOSIS
ORTHÈSE ET PROCÉDÉ DE PRODUCTION D'UNE ORTHÈSE

(30) Priorität: 25.03.2015 DE 102015003819
(43) Veröffentlichungstag der Anmeldung: 27.12.2017
(73) Patentinhaber: EOS GmbH Electro Optical Systems, 82152 Krailling (DE)
(72) Erfinder: KIMM, Martin, 4721 Kelmis (BE); ANTOINE, Vincent, 80634 München (DE)
(74) Vertreter: Prüfer & Partner mbB Patentanwälte · Rechtsanwälte
(86) Internationale Anmeldenummer: PCT/EP2016/056394
(87) Internationale Veröffentlichungsnummer: WO 2016/151020

(56) Entgegenhaltungen:
- WO-A1-2009/015455
- WO-A1-2010/111768
- WO-A1-2013/142343
- US-A1- 2013 226 533

## Beschreibung

Die Erfindung betrifft ein Verfahren zur Herstellung einer Orthese.

Orthesen dienen der Korrektur von Fehlstellungen von Körperteilen wie zum Beispiel Armen, Beinen oder dem Rumpf. Zur Herstellung einer Orthese wurde bisher eine Kopie des betroffenen Körperteils, beispielsweise eines Unterarms, des Patienten angefertigt, indem zunächst ein Gipsabdruck des Körperteils in der gewünschten Korrekturstellung genommen wurde. Danach wurde in einem Tlefzleh- oder Laminiervorgang die Orthesenkontur auf dem Gipsmodell erzeugt, indem eine erwärmte Kunststoffplatte um das Gipsmodel herumgelegt wurde. Anschließend wurde der Kunststoff von der Gipsform in der Schleifwerkstatt entfernt. Der Innere Gipskern wurde dabei beispielsweise durch einen Schlagbohrer mit Meißelaufsatz herausgebrochen und die Kontur der Orthese herausgeschnitten. Nach dem Entfernen des inneren Kernes sowie dem groben Ausschneiden der Spiralkontur mussten die Schnittkanten durch mehrere Schleifvorgänge nachbearbeitet werden. Dies erfolgte erst durch einen groben Schleifvorgang und dann durch finales Feinschleifen bis zur Sollkontur. Weiterhin wurden die Innenflächen zur Tragel<omfortsteigerung mit einer angebrachten Fütterung versehen. Danach konnte die Orthese von einem Orthopädiemeister am Unterarm des Patienten angepasst werden. Stellen, an denen ein erhöhter Druck an der Haut vorlag oder Kanten, die in die Haut einschnitten, konnten nachträglich durch Abschleifen oder mittels lokalen Erhitzens beseitigt werden.

Dieses übliche Herstellungsverfahren weist jedoch eine Reihe von Nachteilen auf. Durch das Gipsabdruckverfahren ist die Geometrie des Modells in der Regel überdimensioniert, wodurch beim Modellieren eine Annäherung an die originalen Maße erwirkt werden muss. Ferner kann es durch die Herstellung per Hand zu groben Fertigungsungenauigkeiten kommen, was zu einer ungleichmäßigen Auflagefläche auf dem Körperteil des Patienten führt. Hierdurch können lokale Druckstellen auf der Haut entstehen, die über einen längeren Zeitraum als besonders unangenehm empfunden werden, bis hin zu einem schmerzvollen Gefühl.

Desweiteren ist der beschriebene Herstellungsprozess aufwendig, da kaum auf Standardkomponenten zurückgegriffen werden kann. Insbesondere durch die notwendige, hohe Individualisierung der einzelnen Produkte sowie der im Vergleich zu anderen industriesparten relativ geringen Stückzahl wird jeder Arbeitsschritt per Hand vorgenommen. Dies erhöht sowohl die Produktionskosten als auch den Zeitaufwand für die Herstellung immens. Parallel hierzu wird die Nachvollziehbarkeit der einzelnen Maßnahmen erschwert.

Da der gesamte Herstellungsprozess händisch erfolgt, kann eine hohe Oberflächengenauigkeit nicht gewährleistet werden. Insbesondere die Anfertigung der Körperteilkopie aus Gips kann deutliche Formabweichungen von mehreren Millimetern verursachen. Bei der nachfolgenden Bearbeitung dieser Gipskopie wird nach dem Erfahrungswissen der Orthopädietechniker zusätzlich Material abgenommen und aufgetragen, um die Oberfläche zu glätten und Abdruckfehler zu beheben. Die ursprüngliche Kontur kann hierbei nur näherungsweise wiederhergestellt werden.

Desweiteren kann eine gleichbleibende Korrekturqualität in der Fertigung nicht gewährleistet werden. In der Regel muss bei der Anprobe der Orthese am Patienten diese nochmals nachkorrigiert werden. Auch ist am Ende des Fertigungsprozesses nicht ersichtlich, ob die zu Beginn durch den Gipsabdruck festgelegte Korrekturstellung genau erreicht werden konnte.

Hauptursache für die auftretenden Fertigungstoleranzen ist somit zum einen die Anfertigung der Körperteitkopie aus Gips, die erhebliche Formabweichungen von mehreren Millimetern erzeugt. Zum anderen sind die darauffolgenden Bearbeitungsschritte der Handschrift des jeweiligen Modellierers zuzuordnen, was sich auf die nachvollziehbare Dokumentation und Fertigungsqualität auswirkt. Das Dokument US-A-2013/226533 offenbart den nächstliegenden Stand der Technik.

Es Ist daher eine Aufgabe der vorliegenden Erfindung, ein verbessertes Verfahren zu entwickeln, wodurch eine Orthese aus definierten Kenngrößen konstruiert, simuliert und hergestellt werden kann. Insbesondere zielt eine solche Verbesserung darauf ab, möglichst viele der oben beschriebenen Probleme des Standes der Technik zu lösen und/oder zu einer höheren Versorgungsqualität bzw. Präzision bzw. einer strukturierten Arbeitsweise und Nachvollziehbarkeit in allen Fertigungsstufen beizutragen.

Die Aufgabe wird durch ein Verfahren zur Herstellung einer Orthese nach Anspruch 1 gelöst. Die Erfindung betrifft auch eine mit diesem Verfahren hergestellte Orthese, die entsprechend aller Unteransprüche des Verfahrens weitergebildet sein kann. Auch das Verfahren kann gemäß den Unteransprüchen zur Orthese weitergebildet sein.

Theoretisch Ist es möglich, Patientendaten des betreffenden Körperteils des Patienten während des Haltens des entsprechenden Körperteils in einer Haltevorrichtung zu gewinnen. Eine solche Haltevorrichtung könnte das Körperteils in einer einstellbaren I<orrekturstellung halten und beispielsweise eine Mehrzahl von Haltebereichen aufweisen sowie eine oder mehrere Korrekturkraftstellvorrichtungen, wobei mindestens eine der Korrekturkraftstellvorrichtungen mit mindestens einem der Haltebereiche verbunden ist. Mittels der Korrekturkraftstellvorrichtungen wären Korrekturkräfte über die mit den I<orrekturl<raftstellvorrichtungen verbundenen Haltebereiche auf den Körperteil ausübbar, und hierdurch der Körperteil in eine Korrekturstellung bringbar.

Dieses Prinzips bedient sich die vorliegende Erfindung bewusst nicht, sondern sie basiert u.a. darauf, dass der entsprechende Körperteil im Wesentlichen haltevorrichtungsfrei gelagert ist. Es wird also keine Haltevorrichtung der oben genannten Art verwendet, "haltevorrichtungsfrei" ist demnach das Gegenteil der Verwendung einer solchen im vorherigen Absatz beschrieben Haltevorrichtung,

Eine Lagerung des entsprechenden Körperteils kann also beispielsweise durch einfaches Auflegen des Körperteils auf eine Oberfläche und/oder durch selbsttätiges Halten des Körperteils durch den Patienten selbst erfolgen. Die gewünschte Position des Körperteils kann dabei durch die Formgebung dieser Oberfläche (die bevorzugt eine gerade Ebene umfasst) und/oder durch willentliche und/oder naturgegebene Körperteilhaltung durch den Patient erzielt werden. Ein Halten des Körperteils dient in diesem Zusammenhang wenn überhaupt nur der temporären ortsfesten Fixierung, etwa in einem medizintechnischen Bildgebungsgerät (s.u.) Nähere beispielhafte Angaben zum möglichen Erreichen der gewünschten Körpertellhaltungen finden sich ebenfalls unten.

Erfindungsgemäß umfasst ein Verfahren zur Herstellung einer Orthese also mindestens folgende Schritte:
- Entgegennahme von Patientendaten mindestens eines Körperteils eines Patienten, wobei der Körperteil bei der Entgegennahme gelagert ist, ohne dass der Körperteil von einer Haltevorrichtung in einer einstellbaren Korrekturstellung gehalten wird,
- Ermittlung und/oder Entgegennahme von Referenzkoordinaten virtueller und/oder physischer Zielobjekte an dem Körperteil, wobei der Körperteil bei der Ermittlung und/oder Entgegennahme gelagert ist, ohne dass der Körperteil von einer Haltevorrichtung in einer einstellbaren Korrekturstellung gehalten wird, und wobei die Zielobjekte mindestens einen für die Anbringung der Orthese am Körperteil repräsentativen Ort an der Oberfläche des Körperteils repräsentieren,
- individuelle Anpassung eines digitalen Orthesenmodells basierend auf den Patientendaten und den Zielkoordinaten,
- Fertigung, insbesondere mindestens teilweise mithilfe eines additiven Fertigungsverfahrens, der Orthese basierend auf dem so angepassten
digitalen Orthesenmodell.

Prinzipiell ist es im Rahmen des erfindungsgemäßen Verfahrens möglich, dass die Patientendaten und/oder die Referenzkoordinaten ganz oder teilweise aus einem medizintechnischen bildgebenden Verfahren, etwa einem MR- bzw. CT-Scan, einer Röntgenbildaufnahme oder ähnlichen medizintechnischen Blldgebungsverfahren ermittelt bzw. abgeleitet werden. Dies gilt einerseits für die Patientendaten und andererseits auch für die Referenzkoordinaten der Zielobjekte. Der entsprechende Körperteil oder gar der gesamte Körper des Patienten werden im Rahmen eines solchen medizintechnischen bildgebenden Verfahrens gelagert und dabei evtl. auch im oben erwähnten Sinne temporär fixiert.

Alternativ oder ergänzend können die Patientendaten und/oder die Referenzkoordinaten ganz oder teilweise dadurch entgegengenommen bzw. ermittelt werden, dass der Patient dazu angeleitet wird, eine bestimmte Haltung des jeweiligen Körperteils einzunehmen und/oder einen vordefinierten Bewegungsablauf durchzuführen. Beispielsweise kann er dazu angeleitet werden, seinen Arm, an den die entsprechende Orthese angepasst werden soll, zu heben und die Finger an der entsprechenden Hand gemäß einem bestimmten Muster in bestimmte Positionen zu bewegen bzw. dort zu halten. Die Position des Arms, also allgemein des Körperteils, und/oder der Finger, allgemein also von Teilbereichen des Körperteils, in die diese jeweils überführt werden sollen, sind bevorzugt Grenzpositionen, also solche Positionen, über die hinaus der Patient den entsprechenden Körperteil bzw. dessen jeweiligen Teilbereich nur noch unter Überwindung von stärkeren körperlichen Widerständen bzw. unter Schmerzen bewegen könnte. Die so gewonnen Patientendaten repräsentieren dann auch Grenzdaten zur Bewegungsfähigkeit des entsprechenden Körperteils bzw. Teilbereichs. Diese Grenzdaten werden ganz bevorzugt in die Anpassung des digitalen Orthesenmodells mit einbezogen.

Als "virtuelle Zielobjekte" werden insbesondere solche Zielobjekte verstanden, die den Patientendaten - etwa als digitaler Zusatzinput - beigestellt werden bzw. wurden. Hierunter zählen beispielsweise Marker, die einen bestimmten Ort bzw. ein bestimmtes Teilobjekt des betreffenden Körperteils markieren und bezeichnen. Eine derartige Beistellung kann manuell, etwa durch einen Behandler oder Befunder, erfolgen, aber auch automatisiert, etwa durch einen automatischen Erkennungsalgorithmus, der auf Basis der Patientendaten eine Objekterkennung (zum Beispiel in Form einer Segmentierung einer bestimmten Körperstruktur) durchführt und entsprechende Marker setzt.

"Physische Zielobjekte" sind solche Objekte, die physisch am betreffenden Körperteil des Patienten angebracht sind. Dabei kann es sich zum Beispiel um Markierungen mit Symbolen, Zeichen o. ä. auf der Oberfläche des Körperteils handeln, etwa aufgemalte Ortsmarkierungen auf der Haut des Patienten, Insbesondere umfassen die Zielobjekte physische Zielobjekte, Insbesondere Fixierungspads, die auf der Oberfläche des Körperteils während eines Scans des Körperteils angebracht sind. Die Patientendaten umfassen dann Scandaten aus diesem Scan. Hierdurch ist gewährleistet, dass die Referenzkoordinaten der physischen Zielobjekte exakt mit den Patientendaten gematcht sind. Fixierungspads können dabei dazu dienen, einen Ort zu definieren, an dem der jeweilige Patient beim Tragen der erfindungsgemäß hergestellten Orthese in Hautkontakt mit der Orthese kommt. Solche Fixierungspads repräsentieren also Polsterungen der Orthese gegenüber der Haut des Patienten, insbesondere die Position und/oder Dimensionierung solcher Polsterungen. Mit anderen Worten: Positionen der Fixierungspads umfassen potenzielle Kontaktpositionen des Körperteils mit der zu fertigenden Orthese.

Weiterhin wird, ohne dass der Körperteil von einer Haltevorrichtung in einer einstellbaren Korrekturstellung gehalten wird, eine Belastungsverteilung aufgrund von vom Körperteil ausgeübten Gegenkräften in Reaktion auf Korrekturkräfte entlang des digitalen Orthesenmodells berechnet und/oder simuliert, wobei die Geometrie der Orthese in dem digitalen Orthesenmodell entsprechend der berechneten und/oder simlulierten Belastungsverteilung optimiert wird und/oder eine Perforations- bzw. Gitterstruktur in dem digitalen Orthesenmodell entsprechend der berechneten bzw. simulierten Belastungsverteilung generiert wird. Eine solche Berechnung bzw. Simulation basiert bevorzugt im Wesentlichen auf einer Auswertung, insbesondere einer mindestens teilautomatisierten Auswertung der betreffenden Patientendaten.

In diesen Zusammenhang ist zu erwähnen, dass die Patientendaten nicht notwendigerweise rein statische Bilddaten im Sinne einer Momentanaufnahme umfassen müssen, sondern auch beispielsweise in Form bewegter Bilddaten, also einer Art Video- oder Ablaufsequenz bereitgestellt werden können. Eine solche Ablaufsequenz repräsentiert dann zum Beispiel bildlich einen Bewegungsablauf des entsprechenden Körperteils, und aus diesem Bewegungsablauf können einfach, insbesondere algorithmusbasiert, bestimmte Bewegungsmuster bzw. Bewegungshemnisse des Körperteils abgeleitet, modelliert und im Endeffekt simuliert werden.

Erfindungsgemäß Ist auch eine Orthese zur Korrektur einer Fehlstellung eines Körperteils, wobei die Orthese mittels eines oben beschriebenen Verfahrens zur Herstellung einer Orthese hergestellt wird.

Ferner kann die so hergestellte Orthese bereichsweise oder vollständig eine perforierte Struktur und/oder eine Gitterstruktur aufweisen, die zumindest bereichsweise entsprechend der berechneten bzw. simulierten Belastungsverteilung angepasst ist. Die Gitterstruktur kann offen oder geschlossen ausgebildet sein. Bei der offenen Gitterstruktur weist die Gitterstruktur Gitteröffnungen auf, die von einem Gitter begrenzt werden. Bei der geschlossenen Gitterstruktur sind die Gitteröffnungen durch eine Materialschicht verschlossen, welche eine geringere oder ein gleiche Materialstärke wie das Gitter aufweist.

Durch die Verwendung einer Gitterstruktur kann die Orthese leichter gefertigt werden bei gleicher Stabilität. Zusätzlich ermöglicht die offene Gitterstruktur eine gute Belüftung eines von der Orthese aufgenommenen Körperteils. Dies erhöht den Tragekomfort der Orthese. Gleichzeitig eröffnet die Verwendung einer offenen Gitterstruktur vielfältige Designoptionen, wodurch wiederum die Akzeptanz der Orthese durch den Patienten verbessert wird.

In einer bevorzugten Ausgestaltung der Orthese weist die Gitterstruktur eine Knochenzellen-Struktur und/oder eine Voronoi-Struktur auf. Diese Strukturen sind besonders stabil bei vergleichsweise geringem Materialaufwand.

Bei der Gitterstruktur kann es sich um ein zweidimensionales Gitter handeln, dass sich In tangentialer Richtung, also parallel zur Oberfläche eines Körperteils erstreckt, wenn die Orthese an dieses angelegt ist. Ferner kann es vorteilhaft sein, wenn die Orthese bereichsweise oder vollständig eine Wandung mit einer oder mehreren Lagen, insbesondere zwei oder drei Lagen, aufweist und die Anzahl der Lagen entlang der Orthese variiert oder konstant ist. In diesem Fall kann es sich bei der Wandung beispielsweise um eine dreidimensionale Gitterstruktur handeln, welche in radialer Richtung, also in einer Richtung, die senkrecht zur Oberfläche eines Körperteils verläuft, wenn an dieses die Orthese angelegt ist, mindestens eine weitere Gitterebene (zweite Lage) aufweist, die sich ebenfalls in tangentialer Richtung erstreckt. Es ist auch denkbar, dass die Gitterstruktur in verschiedenen Bereichen der Orthese eine verschiedene Anzahl an in radialer Richtung angeordneten, in tangentialer Richtung verlaufenden Gitterebenen (Lagen) aufweist. Ferner kann die Anzahl dieser Gitterebenen (Lagen) in tangentialer Richtung der Orthese variieren. Vorzugsweise weist die Wandung der Orthese in stark belasteten Bereichen der Orthese eine größere Anzahl an Lagen auf als in weniger belasteten Bereich der Orthese, Auf diese Weise können in der Orthese weiche Areale ausgebildet werden, um knöcherne Bewegungen bettend zu führen.

Im Folgenden werden ein Beispiel eines erfindungsgemäßen Verfahrens zur Herstellung einer Orthese anhand einer Figur ausführlicher beschrieben. Es zeigt:

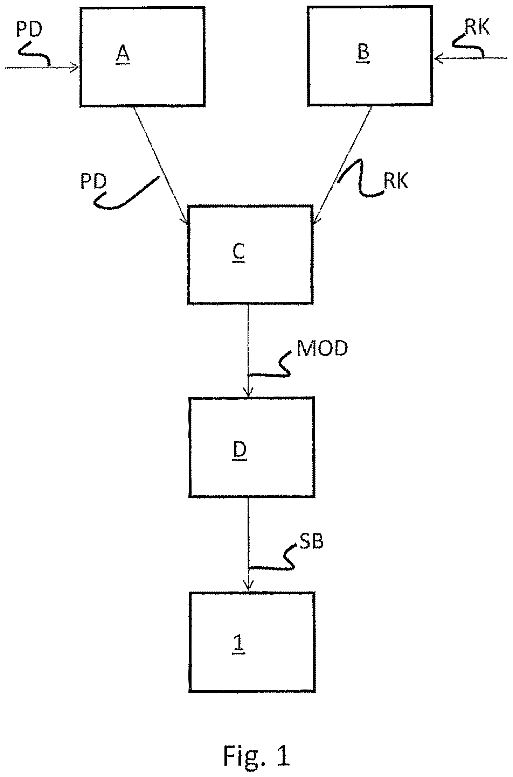

- Fig. 1: ein Ausführungsbeispiel im Blockdiagramm eines erfindungsgemäßen Herstellungsverfahrens,

In einem ersten Schritt A werden Patientendaten PD mindestens eines Körperteils eines Patienten, beispielsweise Patientendaten PD eines Unterarms entgegengenommen. Diese Patientendaten können aus einem praktisch beliebigen bildgebenden Verfahren, insbesondere einem fotografischen oder abtastungsbasierten Scan oder einem medintechnisch bildgebenden Scan stammen. In einem zweiten Schritt B werden Referenzkoordinaten RK virtueller bzw. physischer Zielobjekte an dem Körperteil ermittelt bzw. entgegengenommen. Beide Schritte A und B erfolgen im oben erwähnten Sinne im Wesentlichen haltevorrichtungsfrei.

In einem dritten Schritt C erfolgt eine Individuelle Anpassung eines (beispielsweise bereits in einer idealtypischen Form eines Ausgangsmodells vorliegenden) digitalen Orthesenmodells basierend auf den Patientendaten PD und den Referenzkoordinaten RI<. Beispielsweise kann als Ausgangspunkt der Anpassung des Orthesenmodells aus idealtypischen Formen eines jeweiligen Körperteils eines Kindes, eines weiblichen und eines männlichen Erwachsenen ausgewählt werden (prinzipiell können jedoch auch tierische Patienten berücksichtigt werden, so dass sich die idealtypische Form des Ausgangsmodell dann vorzugsweise auf die jeweilige Spezies, Rasse o.ä. des betreffenden Tieres bezieht). Das Resultat des dritten Schritts C ist ein angepasstes Orthesenmodell MOD, auf dessen Basis nun in einem vierten Schritt D, der zeitlich und/oder räumlich auch von den bisher erfolgten Schritten getrennt erfolgen kann;

Im Schritt D wird, bevorzugt mit einem additiven Fertigungsverfahren die dem angepassten Orthesenmodell entsprechende Orthese 1 für den jeweiligen Körperteil gefertigt.

Auch die Schritte A, B und C sind nicht zwangsläufig räumlich und/oder zeitlich miteinander direkt gekoppelt, sondern können jeweils auch voneinander getrennt erfolgen.

## Patentansprüche

1. Verfahren zur Herstellung einer Orthese (1) zur Korrektur einer Fehlstellung eines Körperteils, umfassend mindestens folgende Schritte:
- Entgegennahme (A) von Patientendaten (PD) mindestens eines Körperteils eines Patienten, wobei der Körperteil bei der Entgegennahme gelagert ist, ohne dass der Körperteil von einer Haltevorrichtung in einer einstellbaren Korrekturstellung gehalten wird,
- Ermittlung und/oder Entgegennahme (B) von Referenzkoordinaten (RK) virtueller und/oder physischer Zielobjekte an dem Körperteil, wobei der Körperteil bei der Ermittlung und/oder Entgegennahme gelagert ist, ohne dass der Körperteil von einer Haltevorrichtung in einer einstellbaren Korrekturstellung gehalten wird, und wobei die Zielobjekte mindestens einen für die Anbringung der Orthese (1) am Körperteil repräsentativen Ort an der Oberfläche des Körperteils repräsentieren,
- individuelle Anpassung (C) eines digitalen Orthesenmodells basierend auf den Patientendaten (PD) und den Referenzkoordinaten (RK),
- Fertigung der Orthese (1) basierend auf dem so angepassten digitalen Orthesenmodell (MOD),
wobei, ohne dass der Körperteil von einer Haltevorrichtung in einer einstellbaren Korrekturstellung gehalten wird, eine Belastungsverteilung aufgrund von vom Körperteil ausgeübten Gegenkräften in Reaktion auf Korrekturkräfte entlang des digitalen Orthesenmodells berechnet und/oder simuliert wird, wobei die Geometrie der Orthese (1) in dem digitalen Orthesenmodell entsprechend der berechneten und/oder simulierten Belastungsverteilung optimiert und/oder eine Perforations- und/oder Gitterstruktur in dem digitalen Orthesenmodell entsprechend der berechneten und/oder simulierten Belastungsverteilung generiert wird.

2. Verfahren gemäß Anspruch 1, wobei , der Schritt der Fertigung (D) mindestens teilweise mithilfe eines additiven Fertigungsverfahrens durchgeführt wird.

3. Verfahren gemäß Anspruch 1 oder 2, wobei die Zielobjekte physische Zielobjekte, insbesondere Fixierungspads, umfassen, die auf der Oberfläche des Körperteils während eines Scans des Körperteils angebracht sind und die Patientendaten Scandaten aus diesem Scan umfassen.

4. Verfahren gemäß Anspruch 3, wobei Positionen der Fixierungspads potenzielle Kontaktpositionen des Körperteils mit der zu fertigenden Orthese (1) umfassen.

5. Orthese (1) zur Korrektur einer Fehlstellung eines Körperteils, hergestellt mittels eines Verfahrens nach einem der Ansprüche 1 bis 4.

6. Orthese nach Anspruch 5, wobei die Orthese eine perforierte Struktur und/oder eine Gitterstruktur aufweist, die zumindest bereichsweise entsprechend der berechneten und/oder simulierten Belastungsverteilung angepasst ist.

7. Orthese nach Anspruch 6, wobei die Gitterstruktur eine Knochenzellen-Struktur und/oder eine Voronoi-Struktur aufweist.

8. Orthese nach Anspruch 6 oder 7, wobei die Orthese bereichsweise oder vollständig eine Wandung mit einer oder mehreren Lagen, insbesondere zwei oder drei Lagen, aufweist und die Anzahl der Lagen entlang der Orthese variiert oder konstant ist.

## Claims

1. A method for producing an orthosis (1) for the correction of a malposition of a body part, comprising at least the following steps:
- receiving (A) patient data (PD) of at least one body part of a patient, wherein during receiving the body part is kept without the body part being held in an adjustable correction position by a holding device,
- determining and/or receiving (B) reference coordinates (RK) of virtual and/or physical target objects on the body part, wherein during determining and/or receiving the body part is kept without the body part being held in an adjustable correction position by a holding device and wherein the target objects represent at least one location on the surface of the body part that is representative for attaching the orthosis (1) on the body part,
- individually adapting (C) a digital orthosis model based on the patient data (PD) and the reference coordinates (RK),
- manufacturing the orthosis (1) based on the digital orthosis model (MOD) that is adapted in this way,
wherein, without the body part being held in an adjustable correction position by a holding device, a load distribution on account of opposing forces exerted by the body part in reaction to correction forces along the digital orthosis model is calculated and/or simulated, and wherein the geometry of the orthosis (1) is optimized in the digital orthosis model in accordance with the calculated and/or simulated load distribution and/or a perforation structure and/or lattice structure in the digital orthosis model is generated in accordance with the calculated and/or simulated load distribution.

2. The method according to claim 1, wherein the step of manufacturing (D) is carried out at least partially using an additive manufacturing method.

3. The method according to claim 1 or 2, wherein the target objects comprise physical target objects, in particular anchoring pads, which are applied to the surface of the body part during a scanning of the body part, and the patient data comprise scan data from this scan.

4. The method according to claim 3, wherein positions of the anchoring pads comprise potential contact positions of the body part with the orthosis (1) to be manufactured.

5. An orthosis (1) for correcting a malposition of a body part, produced by a method according to one of claims 1 to 4.

6. The orthosis according to claim 5, wherein the orthosis comprises a perforated structure and/or a lattice structure which, at least in regions, is adapted in accordance with the calculated and/or simulated load distribution.

7. The orthosis according to claim 6, wherein the lattice structure comprises a bone cell structure and/or a Voronoi structure.

8. The orthosis according to claim 6 or 7, wherein the orthosis comprises, either in regions or everywhere, a wall having one or more layers, in particular two or three layers, and the number of layers varies or is constant along the orthosis.

## Revendications

1. Procédé de fabrication d'une orthèse (1) pour la correction d'un défaut de positionnement d'une partie du corps, comprenant au moins les étapes suivantes :
- réception (A) de données de patient (PD) d'au moins une partie du corps d'un patient, la partie du corps étant montée lors de la réception sans que la partie du corps soit maintenue dans une position de correction réglable par un dispositif de maintien,
- détermination et/ou réception (B) de coordonnées de référence (RK) des objets cibles virtuels et/ou physiques sur la partie du corps, la partie du corps étant positionnée lors de la détermination et/ou réception sans que la partie du corps soit maintenue dans une position de correction réglable par un dispositif de maintien, et les objets cibles représentant au moins un emplacement représentatif de la mise en place de l'orthèse (1) sur la partie du corps à la surface de la partie du corps,
- adaptation individuelle (C) d'un modèle d'orthèse numérique sur la base des données de patient (PD) et des coordonnées de référence (RK),
- fabrication de l'orthèse (1) sur la base du modèle d'orthèse numérique (MOD) ainsi adapté,
dans lequel, sans que la partie du corps soit maintenue dans une position de correction réglable par un dispositif de maintien, une répartition de contrainte due à des forces antagonistes exercées par la partie du corps en réaction à des forces de correction est calculée et/ou simulée le long du modèle d'orthèse numérique, dans lequel la géométrie de l'orthèse (1) est optimisée dans le modèle d'orthèse numérique selon la répartition de contrainte calculée et/ou simulée et/ou une structure de perforation et/ou de grille est générée dans le modèle d'orthèse numérique selon la répartition de contrainte calculée et/ou simulée.

2. Procédé selon la revendication 1, dans lequel l'étape de fabrication (D) est réalisée au moins en partie à l'aide d'un procédé de fabrication additive.

3. Procédé selon la revendication 1 ou 2, dans lequel les objets cibles comprennent des objets cibles physiques, en particulier des tampons de fixation, qui sont mis en place sur la surface de la partie du corps pendant un balayage de la partie du corps, et les données de patient comprennent des données de balayage provenant de ce balayage.

4. Procédé selon la revendication 3, dans lequel les positions des tampons de fixation comprennent des positions de contact potentielles de la partie du corps avec l'orthèse (1) à fabriquer.

5. Orthèse (1) pour la correction d'un défaut de positionnement d'une partie du corps, fabriquée au moyen d'un procédé selon l'une quelconque des revendications 1 à 4.

6. Orthèse selon la revendication 5, dans laquelle l'orthèse présente une structure perforée et/ou une structure de grille, qui est adaptée au moins en partie selon la répartition de contrainte calculée et/ou simulée.

7. Orthèse selon la revendication 6, dans laquelle la structure de grille présente une structure de cellules osseuses et/ou une structure de Voronoi.

8. Orthèse selon la revendication 6 ou 7, dans laquelle l'orthèse présente en partie ou en totalité une paroi avec une ou plusieurs couches, en particulier deux ou trois couches, et le nombre de couches le long de l'orthèse varie ou est constant.
